# FASCICULE DE BREVET EUROPEEN

(11) **EP 0 865 775 B1**
(45) Date de publication et mention de la délivrance du brevet: **02.01.2003**
(21) Numéro de dépôt: 98420050.1
(22) Date de dépôt: 19.03.1998
(51) Int. Cl.: A61F 2/34

(54) **Implant en polyéthylène à billes de repérage**
Polyäthylenprothese mit Markierungskugeln
Polyethylene implant with marker balls

(30) Priorité: 19.03.1997 FR 9703574
(43) Date de publication de la demande: 23.09.1998
(73) Titulaire: Tornier SA, 38330 Saint Ismier (FR)
(72) Inventeur: Oudard Jean-Luc, 38330 Saint Nazaire Les Eymes (FR)
(74) Mandataire: Schmitt, John

(56) Documents cités:
- FR-A- 2 720 930
- GB-A- 2 134 360
- US-A- 4 883 490
- US-A- 4 892 548

## Description

La présente invention a trait à un implant acétabulaire ou cotyloïdien destiné à être placé dans une cavité articulaire endommagée pour coopérer notamment avec la tête d'une prothèse fémorale, l'ensemble constituant une prothèse totale de hanche.

On connaît des implants acétabulaires, réalisés entièrement en matière plastique telle que du polyéthylène, qui sont destinés à coopérer avec une tête fémorale naturelle ou artificielle dans une arthroplastie de la hanche. De tels implants sont mis en place en utilisant un ciment qui solidarise lesdits implants avec la cavité osseuse endommagée.

Toutefois, de tels implants présentent des inconvénients en ce qui concerne leur visibilité par radiographie et par conséquent l'impossibilité pour le chirurgien de vérifier deux positions géométriques importantes qui sont les suivantes :
- la position axiale et l'enfoncement de l'implant par rapport à la cavité osseuse du patient,
- l'usure de l'implant et l'enfoncement de la boule ou tête de la prothèse fémorale dans l'implant.

En outre, il est également impossible pour le chirurgien de mesurer la taille de l'implant à l'intérieur de la cavité osseuse.

On connaît d'après le document US 4 892 548 des implants qui comportent deux fils de cerclage, réalisés dans une matière métallique, et qui sont disposés sur la périphérie de l'implant afin que le premier fil soit placé suivant une position équatoriale, tandis que le second est positionné suivant un méridien.

On constate que ce système de cerclage est onéreux et délicat car il nécessite d'une part des opérations de fraisage et d'ébavurage de la surface extérieure de l'implant en polyéthylène et d'autre part un montage difficile des fils dans leur logement.

En outre le positionnement des fils métalliques sur la périphérie de l'implant ne permet pas de connaître les positions géométriques définies ci-dessus et plus particulièrement l'usure et l'enfoncement de la boule appartenant à la prothèse fémorale.

Les perfectionnements qui font l'objet de la présente invention visent à remédier aux inconvénients des implants réalisés avec des fils de cerclage afin de mieux répondre aux pratiques actuelles de mise en place des implants.

On connaît d'après le document US 4 883 490 des implants qui comportent un fil de cerclage réalisé dans une matière métallique disposé sur la périphérie de l'implant suivant une position équatoriale et un plot radio opaque introduit au niveau du pôle de l'implant.

On connaît d'après le document FR 2 720 930 un insert d'implant cotyloïdien comportant sur une partie de sa couronne périphérique un rebord anti-luxation dans lequel sont fixés des éléments radio opaques.

Ces éléments ont pour objet le repérage du bord anti-luxation de l'implant afin de pouvoir contrôler sa position et plus particulièrement sa rotation.

De ce fait la position de plots sur le bord anti-luxation de l'implant ne permet pas de vérifier les deux positions géométriques importantes décrites ci-dessus.

A cet effet, l'implant acétabulaire ou cotyloïdien en polyéthylène suivant la présente invention comprend une face externe à profil courbe ou en portion de sphère, dont le diamètre est prévu pour assurer sa fixation dans la cavité osseuse, un logement interne présentant une forme en portion de sphère destiné à recevoir une boule ou tète sphérique d'une prothèse, et des éléments radio opaques fixés dans l'implant pour son repérage radiographique, lesdits éléments étant constitués de billes en acier dont quatre sont également réparties sur la circonférence de plus grand diamètre de l'implant et une sur le pôle pour permettre la vérification, d'une part de la position axiale et de l'enfoncement de l'implant par rapport à la cavité osseuse et d'autre part l'usure de l'implant et l'enfoncement de la boule de la prothèse fémorale dans ledit implant.

L'implant acétabulaire ou cotyloïdien suivant la présente invention comprend une face externe en portion de sphère qui comporte des trous borgnes dans lesquels sont introduites en force les billes.

L'implant acétabulaire ou cotyloïdien suivant la présente invention comporte une circonférence de plus grand diamètre qui est prévue dans une zone formant une ceinture périphérique se trouvant à la jointure entre la face externe et une face externe à profil plan de l'implant.

L'implant acétabulaire ou cotyloïdien suivant la présente invention comporte des billes qui sont également réparties tous les 90° degrés sur la circonférence de plus grand diamètre.

L'implant acétabulaire ou cotyloïdien suivant la présente invention comporte des billes qui sont réalisées en acier inox ou tout autre alliage.

De plus, la face externe de l'implant comporte des trous borgnes pour enchâsser en force les billes et les maintenir dans leurs positions.

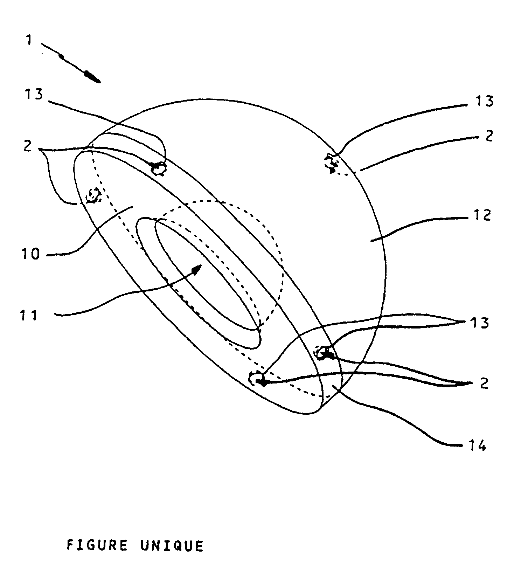

La description qui va suivre en regard du dessin annexé, donné à titre d'exemple non limitatif, permettra de mieux comprendre l'invention, les caractéristiques qu'elle présente et les avantages qu'elle est susceptible de procurer :
La figure unique est une vue en perspective de l'implant en polyéthylène suivant la présente invention.

On a représenté un implant 1 qui est réalisé sous la forme d'un moulage d'une matière plastique telle que du polyéthylène affectant la forme extérieure d'une portion de sphère dont le diamètre est prévu pour en assurer sa fixation dans le cotyle osseux.

L'implant 1 est creusé au milieu de sa face externe 10 à profil plan d'un logement 11 présentant la forme d'une portion de sphère qui est destiné à recevoir, par exemple, la boule ou tête d'une prothèse fémorale non représentée.

La face externe 12 à profil courbe de l'implant 1 est traitée, de sorte qu'elle soit apte à adhérer correctement au ciment chirurgical habituellement utilisé pour permettre directement la fixation dudit implant dans le cotyle osseux.

La face externe 12 est percée de trous borgnes 13 dont au moins quatre sont ménagés et également répartis sur la circonférence de plus grand diamètre de l'implant 1. En effet, on entend par circonférence de plus grand diamètre une zone 14 formant une ceinture périphérique se trouvant à la jointure entre les faces externes à profil courbe 12 et externes à profil plan 10.

Les trous borgnes 13 disposés sur l'équateur sont prévus pour recevoir en force des billes 2 réalisées, par exemple, en acier inox ou tout autre alliage.

Un autre trou borgne 13 est percé sur le pôle de l'implant 1 et dans lequel est enchâssée une autre bille 2 en acier inox.

Par exemple, les billes 2 disposées sur la circonférence de plus grand diamètre de l'implant 1 sont également réparties et plus particulièrement tous les 90° degrés.

On constate que les cinq billes 2 placées sur la face externe 12 apparaîtront clairement lors de prises de radiographie.

L'emplacement particulier des cinq billes 2 sur la face externe 12 permet au chirurgien d'effectuer les mesures et les contrôles suivants :
- si les quatre billes équatoriales 2 sont visibles à la radio, le chirurgien pourra relier deux à deux les billes opposées par une droite. L'intersection des deux droites donnera le centre de l'implant 1.
- si une ou deux billes équatoriales sont masquées, le milieu des deux billes extrêmes 2 donne également le centre de l'implant 1
- en reliant le centre de l'implant 1 à la bille polaire 2, le chirurgien obtient l'axe de l'implant 1.
- après avoir déterminé le centre de la boule ou tête de la prothèse, le chirurgien pourra comparer le centre théorique de l'implant 1 et mesurer la direction et la valeur de l'usure. Sachant que l'échelle est donnée par la comparaison de la mesure des deux billes équatoriales extrêmes 2 avec le diamètre réel de la sphère sur laquelle sont disposées les billes.

Dans ces conditions l'implant 1 suivant la présente invention permet aux chirurgiens d'effectuer les mesures et contrôles nécessaires de manière bien plus satisfaisante que les méthodes actuelles et imprécises.

## Revendications

1. Implant acétabulaire ou cotyloïdien en polyéthylène destiné à être placé dans une cavité osseuse, comprenant une face externe (12) à profil courbe ou en portion de sphère, dont le diamètre est prévu pour assurer sa fixation dans la cavité osseuse, un logement interne (11) présentant une forme en portion de sphère destinée à recevoir une boule ou tête sphérique d'une prothèse, et des éléments (2) radio opaques fixés dans l'implant (1) pour son repérage radiographique, **caractérisé en ce qu'**il comprend sur sa face externe (12) entant qu'éléments radio opaques des billes (2) en acier dont quatre sont également réparties sur la circonférence de plus grand diamètre de l'implant (1) et une sur le pôle pour permettre la vérification, d'une part de la position axiale et de l'enfoncement de l'implant (1) par rapport à la cavité osseuse et d'autre part l'usure de l'implant et l'enfoncement de la boule de la prothèse fémorale dans ledit implant.

2. Implant suivant la revendication 1, **caractérisé en ce que** la face externe (12) en portion de sphère comporte des trous borgnes (13) dans lesquels sont introduites en force les billes (2).

3. Implant suivant la revendication 1, **caractérisé en ce que** la circonférence de plus grand diamètre est prévue dans une zone (14) formant une ceinture périphérique se trouvant à la jointure entre ladite face externe (12) et une face externe à profil plan (10) de l'implant (1).

4. Implant suivant la revendication 2, **caractérisé en ce que** les billes (2) disposées dans les trous borgnes (13) prévus sur la circonférence de plus grand diamètre sont également réparties tous les 90° degrés.

5. Implant suivant la revendication 1, **caractérisé en ce que** les billes (2) sont réalisées en acier inox ou tout autre alliage.

## Patentansprüche

1. Polyethylenimplantat für Hüftgelenkpfannen oder Gelenkpfannen, das dazu bestimmt ist, in den Hohlraum eines Knochens eingesetzt zu werden, umfassend eine äußere Oberfläche (12) mit einem gekrümmten oder kugelsektorförmigen Profil, wobei der Durchmesser so ausgelegt wird, dass dessen Befestigung in dem Hohlraum des Knochens sichergestellt wird, eine innere Lagerung (11), welche die Form eines Kugelsektors aufweist und dazu bestimmt ist, eine Kugel oder einen kugelförmigen Kopf einer Prothese aufzunehmen, und röntgenstrahlungsundurchlässige Elemente (2), die in dem Implantat (1) für dessen radiographische Ausrichtung befestigt sind,
**dadurch gekennzeichnet, dass** es auf seiner äußeren Oberfläche (12) als röntgenstrahlungsundurchlässige Elemente Stahlkugeln (2) umfasst, wobei vier in gleichmäßigen Abständen längs der Umfangslinie mit dem größten Durchmesser des Implantats (1) und eine auf dem Pol angeordnet sind, um einerseits die axiale Position und die Einsenkung des Implantats (1) in Bezug auf den Hohlraum des Knochens und andererseits die Abnutzung des Implantats und die Einsenkung der Kugel der Oberschenkelprothese in das Implantat überprüfen zu können.

2. Implantat nach Anspruch 1, **dadurch gekennzeichnet, dass** die äußere, kugelsektorförmige Oberfläche (12) Blindbohrungen (13) umfasst, in die die Kugeln (2) mit großer Kraft eingeführt werden.

3. Implantat nach Anspruch 1, **dadurch gekennzeichnet, dass** die Umfangslinie mit dem größten Durchmesser in einer Zone (14) vorgesehen ist, die einen peripher verlaufenden Gürtel bildet, der sich an der Verbindung zwischen dieser äußeren Oberfläche (12) und einer äußeren Oberfläche (10) mit ebenem Profil des Implantats (1) befindet.

4. Implantat nach Anspruch 2, **dadurch gekennzeichnet, dass** die Kugeln (2), die in den Blindbohrungen (13) angeordnet sind, welche auf der Umfangslinie mit dem größten Durchmesser vorgesehen sind, gleichmäßig im Abstand von jeweils 90° verteilt sind.

5. Implantat nach Anspruch 1, **dadurch gekennzeichnet, dass** die Kugeln (2) aus nichtrostendem Stahl oder jeder anderen Legierung hergestellt sind.

## Claims

1. Acetabular or cotyloid implant of polyethylene intended to be placed in a bone cavity, comprising an outer face (12) of curved profile or in the form of a portion of a sphere, the diameter of which is intended to ensure its fixing in the bone cavity, an inner recess (11) having the form of a portion of a sphere intended to receive a ball or spherical head of a prosthesis, and radiopaque elements (2) fixed in the implant (1) for its radiographic marking, **characterized in that** it comprises on its outer face (12), as radiopaque elements, steel balls (2), four of which are distributed equally on the circumference of greater diameter of the implant (1) and one of which is at the pole in order to permit verification, on the one hand, of the axial position and extent of insertion of the implant (1) relative to the bone cavity, and, on the other hand, the wear of the implant and the extent of insertion of the head of the femoral prosthesis in said implant.

2. Implant according to Claim 1, **characterized in that** the outer face (12) in the form of a portion of a sphere comprises blind holes (13) in which the balls (2) are introduced with force.

3. Implant according to Claim 1, **characterized in that** the circumference of greater diameter is provided in a zone (14) forming a peripheral belt situated at the join between said outer face (12) and an outer face of plane profile (10) of the implant (1).

4. Implant according to Claim 2, **characterized in that** the balls (2) arranged in the blind holes (13) provided on the circumference of greater diameter are distributed equally at every 90 degrees.

5. Implant according to Claim 1, **characterized in that** the balls (2) are made of stainless steel or any other alloy.
